# EUROPÄISCHE PATENTSCHRIFT

(11) **EP 2 242 738 B1**
(45) Veröffentlichungstag und Bekanntmachung des Hinweises auf die Patenterteilung: **28.03.2012**
(21) Anmeldenummer: 09712743.5
(22) Anmeldetag: 17.02.2009
(51) Int. Cl.: C07C 243/38, A61K 31/166

(54) **SGK1-INHIBITOREN ZUR PROPHYLAXE UND/ODER THERAPIE VON VIRALEN ERKRANKUNGEN UND/ODER KARZINOMEN**
SGK1 INHIBITORS FOR THE PROPHYLAXIS AND/OR THERAPY OF VIRAL DISEASES AND/OR CARCINOMAS
INHIBITEURS DE SGK1 UTILISÉS POUR LA PROPHYLAXIE ET/OU LA THÉRAPIE D'INFECTIONS VIRALES ET/OU DE CARCINOMES

(30) Priorität: 18.02.2008 DE 102008010361
(43) Veröffentlichungstag der Anmeldung: 27.10.2010
(73) Patentinhaber: Merck Patent GmbH, 64293 Darmstadt (DE)
(72) Erfinder: FUCHSS, Thomas, 64625 Bensheim-Auerbach (DE); GERICKE, Rolf, 64342 Seeheim-Jugenheim (DE); BEIER, Norbert, 64354 Reinheim (DE); LANG, Florian, 72076 Tuebingen (DE); LANG, Philipp, 72076 Tuebingen (DE); LANG, Karl, 72076 Tuebingen (DE)
(86) Internationale Anmeldenummer: PCT/EP2009/001099
(87) Internationale Veröffentlichungsnummer: WO 2009/103484

(56) Entgegenhaltungen:
- WO-A-96/17840
- WO-A-2005/037773
- WO-A-2007/093264

## Beschreibung

Die vorliegende Erfindung betrifft Verbindungen, welche SGK1 (serum and glucocorticoid dependent kinase 1) hemmen, zur therapeutischen Behandlung von viralen Erkrankungen und/oder Karzinomen.

Die Serum- und Glucocorticoid-abhängige Kinase-1 (SGK1, serum and glucocorticoid dependent kinase1) ist ein in eukaryotischen Zellen ubiquitär vorkommendes Protein. SGK1 steht dabei unter der Kontrolle von zellulärem Stress, wie beispielsweise Zellschrumpfung, und Hormonen, wie beispielsweise Gluco- und Mineralcorticoiden. SGK1 wird durch Insulin und Wachstumsfaktoren über Phosphatidylinositol-3-Kinase und PDK1 (3-Phosphoinositide dependent kinase-1) aktiviert.

SGK1 wurde ursprünglich aus Rattenmammakarzinomazellen kloniert (Webster MK, Goya L, Firestone GL. J. Biol. Chem. 268 (16): 11482-11485, 1993; Webster MK, Goya L, Ge Y, Maiyar AC, Firestone GL. Mol. Cell. Biol. 13 (4): 2031-2040, 1993). Die humane Kinase hSGK wurde als zellvolumenreguliertes Gen aus Leberzellen kloniert (Waldegger S, Barth P, Raber G, Lang F. Proc. Natl. Acad. Sci. USA 94: 4440-4445, 1997). Es zeigte sich, daß die Rattenkinase (Chen SY, Bhargava A, Mastroberardino L, Meijer OC, Wang J, Buse P, Firestone GL, Verrey F, Pearce D. Proc. Natl. Acad. Sci. USA 96: 2514-2519, 1999; Naray-Fejes-Toth A, Canessa C, Cleaveland ES, Aldrich G, Fejes-Toth G. J. Biol. Chem. 274: 16973-16978, 1999) den epithelialen Na⁺-Kanal (ENaC) stimuliert. Weiter wurde gezeigt, daß eine gesteigerte Aktivität des ENaC mit Hypertonie einhergeht (Warnock DG. Kidney Ind. 53 (1): 1824, 1998).

Die hSGK wird auch im Gehirn exprimiert (Waldegger S, Barth P, Raber G, Lang F. Proc. Natl. Acad. Sci. USA 94: 4440-4445, 1997), wo sie die spannungsabhängigen K⁺-Kanäle Kv1.3 reguliert. Es wurde gezeigt, daß diese K⁺-Kanäle des Kv1.3-Typs involviert sind in der Regulation der neuronalen Erregbarkeit (Pongs O. Physiol. Rev. 72: 69-88, 1992), der Regulation von Zellproliferation (Cahalan MD und Chandy KG. Cur. Opin. Biotech. 8 (6): 749-756, 1997) sowie der Regulation von apoptotischem Zelltod (Szabo 1, Gulbin E, Apfel H, Zhan X, Barth P, Busch AE, Schlottmann K, Pongs O Lang F. J. Biol. Chem. 271: 20465-20469, 1999; Lang F, Szabo I, Lepple-Wienhues A, Siemen D, Gulbins E. News Physiol. Sci. 14: 194-200, 1999). Kv1.3 ist ferner wichtig bei der Regulation der Lymphozytenproliferation und -funktion (Cahalan MD und Chandy KG, Cur. Opin. Biotech. 8 (6): 749-756, 1997). Es wurden zwei weitere Mitglieder der SGK-Familie kloniert, die SGK2 und SGK3 (Kobayashi T, Deak M, Morrice N, Cohen P. Biochem. J. 344: 189-197, 1999). Ferner zeigte sich, dass die SGK eine Serin-Threonin-Proteinkinase-Familie bilden, die transkriptionell und posttranskriptionell reguliert werden können. Wie die SGK1, werden auch die SGK2 und SGK3 durch z. B. Insulin und IGF1 über den PI3-Kinase-Weg aktiviert. Eine vollständige Charakterisierung der SGK Proteinfamilie hat aber bis jetzt nicht stattgefunden.

In der Zwischenzeit ist SGK1 auch als Target für diverse therapeutische und diagnostische Anwendungen bekannt.

In der DE 197 08 173 konnte beispielsweise gezeigt werden, daß die hSGK1 bei vielen Erkrankungen, die durch Zellvolumenänderung pathophysiologisch beeinflußt werden, wie beispielsweise Hypernatriämie, Hyponatriämie, Diabetes mellitus, Niereninsuffizienz, Hyperkatabolismus, hepatische Encephalopathie und mikrobielle oder virale Infektionen, ein beträchtliches diagnostisches Potential besitzt.

In der DE 199 17 990 sind Kinase-Hemmstoffe, wie beispielsweise Staurosporin, Chelerythrin oder transdominant inhibitorische Kinase beschrieben worden, die bei der Therapie zellvolumenabhängiger Erkrankungen eingesetzt werden können.

Darüber hinaus ist aus der WO 2004/079003 A1 die Verwendung von SGK1 als diagnostisches und therapeutisches Target für Koagulopathien, Angiopathien, pulmonale Hypertonie und Arteriosklerose bekannt.

Die Rolle von SGK1 bei der Entstehung von Tumoren wird in der Fachliteratur dagegen sehr kontrovers und widersprüchlich diskutiert.

Die Erfindung stellt sich nun die Aufgabe, weitere neue therapeutische und diagnostische Anwendungsgebiete unter Verwendung von SGK1 als Zielmolekül (Target) bereitzustellen.

Gegenstand der Erfindung sind auch die optisch aktiven Formen (Stereoisomeren), die Enantiomeren, die Racemate, die Diastereomeren sowie die Hydrate und Solvate der Verbindungen der Formeln Ia und Ib. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Gegenstand der Erfindung sind die Verbindungen der Enantiomere der Formel Ia (S-Konfiguration) und Formel Ib (R-Konfiguration): 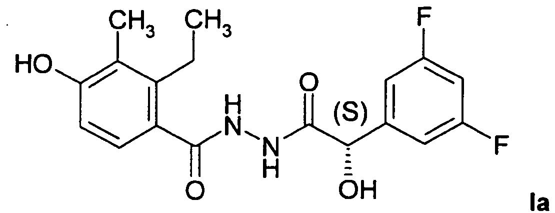 und 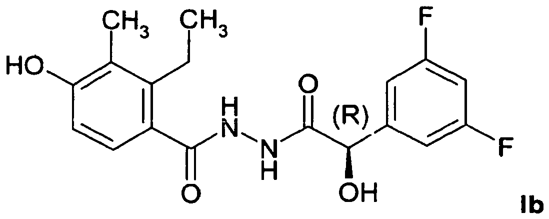 sowie ihre pharmazeutisch verwendbaren Tautomere, Salze, Stereoisomere und die Enantiomeren, einschließlich deren Mischungen in allen Verhältnissen.

Bei den in Formeln la und Ib dargestellten Verbindung handelt es sich um 2-Ethyl-4-hydroxy-3-methyl-benzoesäure-N'-[2-(3,5-difluor-phenyl)-2-hydroxyacetyl]-hydrazid. Vorzugsweise werden diesee Verbindungen als Arzneimittel verwendet. Besonders bevorzugt werden die durch die Formeln Ia und Ib dargestellte Verbindung zur Prophylaxe und/oder Behandlung von viraler Hepatitis eingesetzt. Überraschenderweise konnte nämlich festgestellt werden, dass es sich bei den Verbindung gemäß der Formeln Ia und Ib um Inhibitoren von SGK1 handelt. Des Weiteren konnte erfolgreich nachgewiesen werden, dass sich besagten Verbindungen zur Therapie von viralen Erkrankungen und karzinomatösen Erkrankungen eignebn.

Bezüglich weiterer Eigenschaften und Merkmale sowie der Herstellung von Vertretern dieser Verbindungsklasse wird auf die WO 2007/093264 A1 der Anmelderin Bezug genommen, deren Offenbarungsgehalt vollständig durch ausdrückliche Bezugnahme zum Inhalt der vorliegenden Beschreibung gemacht wird.

Die viralen Erkrankungen sind vorzugsweise aus der Gruppe lymphozytäre Choriomeningitis, virale Hepatitis, virale Myocarditis, Aids, Herpes, Papilloma und virale Lungenentzündung ausgewählt. Unter Herpes und Papilloma sollen im Sinne der vorliegenden Erfindung Infektionen bzw. Infektionserkrankungen verstanden werden, die durch Herpes- bzw. Papilloma-Viren verursacht werden.

Die Karzinome sind bevorzugt aus der Gruppe Colonkarzinom, Mammakarzinom, Magenkarzinom und Lungenkarzinom ausgewählt.

Weiterhin können die Verbindungen gemäß der Formeln Ia und Ib in Form von pharmazeutischen Formulierungen vorliegen. Derartige pharmazeutische Formulierungen können zur Verabreichung über einen beliebigen geeigneten Weg, beispielsweise auf oralem, rektalem, nasalem, topischem, vaginalem oder parenteralem Wege, angepasst werden. Derartige Formulierungen können mit Hilfe auf dem pharmazeutischen Gebiet bekannten Verfahren hergestellt werden, indem beispielsweise die besagten Verbindungen mit dem bzw. den Trägerstoff(en) oder Hilfsstoff(en) zusammengebracht werden.

Bezüglich weiterer Merkmale und Einzelheiten zu den pharmazeutischen Formulierungsmöglichkeiten der Verbindungen gemäß Formel II wird ebenfalls auf die WO 2007/093264 A1 verwiesen.

Eine therapeutisch wirksame Menge einer Verbindung der vorliegenden Erfindung hängt von einer Reihe von Faktoren ab, einschließlich z.B. dem Alter und Gewicht des Menschen oder Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung im allgemeinen im Bereich von 0,1 bis 1000 mg/kg Körpergewicht des Empfängers (Säugers) pro Tag, im besonderen im Bereich von 0,1 bis 100 mg/kg Körpergewicht pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so dass die Gesamttagesdosis die gleiche ist. Eine wirksame Menge eines Salzes oder Solvats oder eines physiologisch funktionellen Derivats davon kann als Anteil der wiksamen Menge der erfindungsgemäßen Verbindung per se bestimmt werden. Es lässt sich annehmen, dass ähnliche Dosierungen für die Behandlung der anderen, oben erwähnten Krankheitszustände geeignet sind.

Die vorliegende Erfindung betrifft auch die Verwendung von Verbindungen gemäß der Formeln Ia und ib zur Herstellung eines Arzneimittels, zur Prophylaxe und/oder Behandlung von viralen Erkrankungen und/oder Karzinomen. Bezüglich weiterer Merkmale und Einzelheiten wird auf die bisherige Beschreibung verwiesen.

Die Erfindung betrifft schließlich auch ein Verfahren zur Prophylaxe und/oder Behandlung von viralen Erkrankungen und/oder Karzinomen, umfassend die Verabreichung einer therapeutisch wirksamen Menge von Verbindungen gemäß der Formeln Ia und Ib. Der Ausdruck "therapeutisch wirksame Menge" bedeutet dabei eine Menge von besagten Verbindungen bzw. darauf basierenden Arzneimitteln oder Pharmazeutika, welche, verglichen zu einem entsprechenden Patienten, der diese Menge nicht erhalten hat, folgendes bewirkt: verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung. Bezüglich weiterer Merkmale und Einzelheiten wird ebenfalls auf die bisherige Beschreibung Bezug genommen.

Weiterhin betrifft die vorliegende Erfindung auch die in der WO 2006/105850 A1 der Anmelderin beschriebenen und durch die Formeln la und Ib dargestellten Verbindungen zur Prophylaxe und/oder Behandlung von viralen Erkrankungen und/oder Karzinomen. Bezüglich weiterer Eigenschaften und Merkmale sowie der Herstellung dieser Verbindungen wird auf die WO 2006/105850 A1 der Anmelderin Bezug genommen, deren Offenbarungsgehalt vollständig durch ausdrückliche Bezugnahme zum Inhalt der vorliegenden Beschreibung gemacht wird.

Die bestehenden und weitere Merkmale der Erfindung ergeben sich aus der nachfolgenden Beschreibung von bevorzugten Ausführungsformen in Verbindung mit den Unteransprüchen und den Figuren. Hierbei können die einzelnen Merkmale jeweils für sich oder zu mehreren in Kombination miteinander verwirklicht sein.

In den Abbildungen zeigen:
- Figur 1:: eine gesteigerte CD8⁺ T-Zellantwort gegen lymphozytäres Choriomeningitis-Virus (LCMV) in SGK1-Knockout-Mäusen,
- Figur 2:: virale Replikation von LCMV in verschiedenen Geweben bei SGK1-Knockout-Mäusen und SGK1-Wildtyp-Geschwistermäusen,
- Figur 3:: das Ergebnis einer immunohistochemischen Untersuchung auf LCMV-Antigen bei SGK1-Knockout-Mäusen und Wildtyp-Geschwistermäusen,
- Figur 4:: den Einfluss von SGK1 auf den Verlauf von viraler Hepatitis,
- Figur 5:: Wirkung eines SGK1-Inhibitors auf den Verlauf einer viralen Hepatitis,
- Figur 6:: die Entwicklung von Colonkarzinomen bei SGK1-Knockout Mäusen im Vergleich zu SGK1-Wildtyp-Mäusen,
- Figur 7:: die Hemmung von Colonkarzinomen durch einen SGK1-Inhibitor,

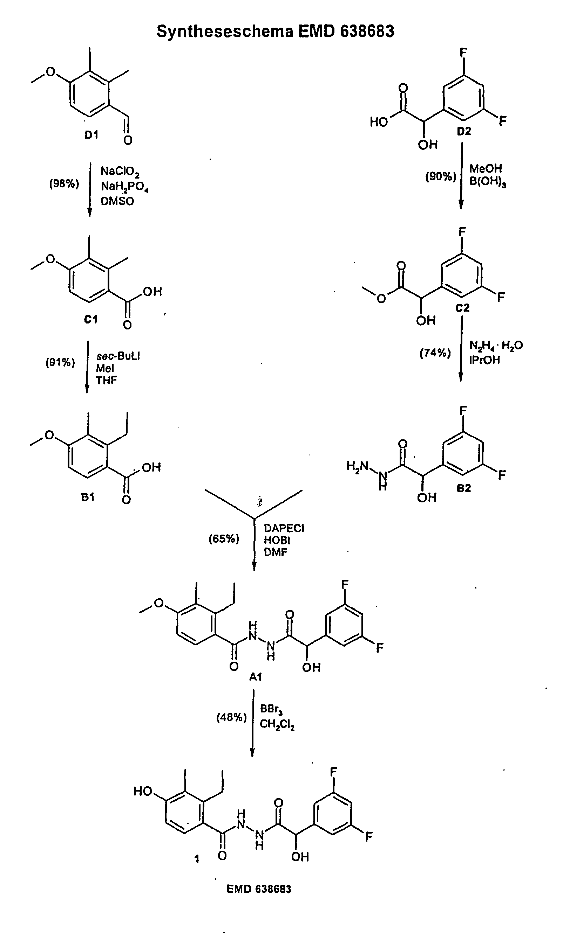

- Figur 8:: ein Syntheseschema des SGK1-Inhibitors 2-Ethyl-4-hydroxy-3-methyl-benzoesäure-N'-[2-(3,5-difluor-phenyl)-2-hydroxy-acetyl]-hyrazid I (EMD 638683)

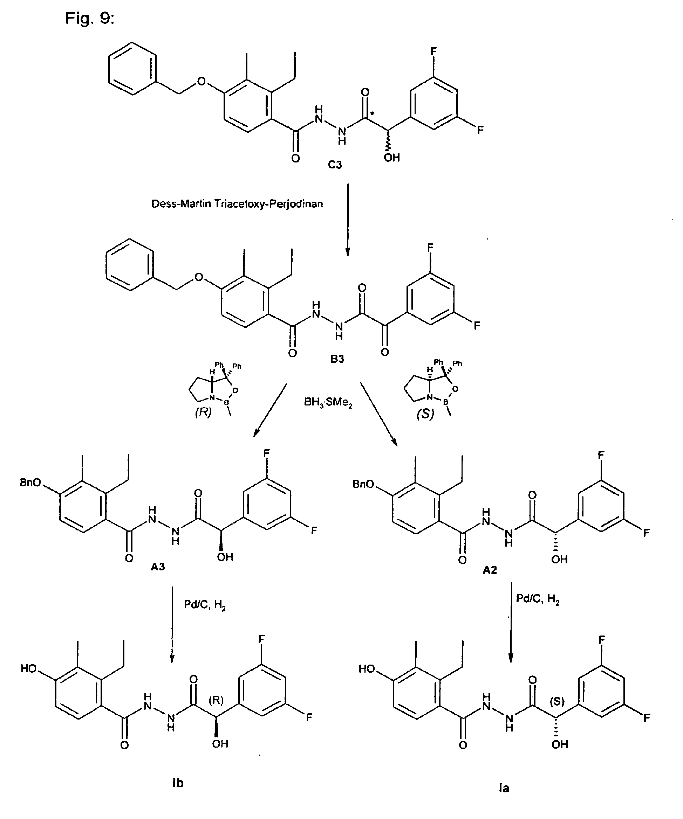

- Figur 9:: ein Syntheseschema der enantiomeren SGK1-Inhibitoren 2-Ethyl-4-hydroxy-3-methyl-benzoesäure-N'-[(S)-2-(3,5-difluor-phenyl)-2-hydroxy-acetyl]-hydrazid (la) 2-Ethyl-4-hydroxy-3-methyl-benzoesäure-N'-[(R)-2-(3,5-difluor-phenyl)-2-hydroxy-acetyl]-hydrazid (Ib)

### Beispiele

### I. 2-Ethyl-4-hydroxy-3-methyl-benzoesäure-N'-[2-(3,5-difluor-phenyl)-2-hydroxyacetyl]-hydrazid (EMD 638683)

Es werden 70.0 g von 2-Ehtyl-4-methoxy-3-methyl-benzoesäure-N'-[2-(3,5-difluor-phenyl)-2-hydroxy-acetyl]-hydrazid (A1) in 350 ml Dichlormethan suspendiert. Danach werden 105 ml Bortribromid tropfenweise hinzugegeben. Die Reaktionsmischung wird bei Raumtemperatur während 3 Stunden gerührt. Anschließend wird die erhaltene Lösung vorsichtig in einen Liter Eiswasser dekantiert. Die wässrige Phase wird zweimal mit 500 ml Ethylacetat extrahiert. Die vereinigten organischen Phasen werden danach einmal mit 300 ml Wasser extrahiert, über Natriumsulfat getrocknet und anschließend filtriert. Das Filtrat wird im Vakuum konzentriert. Danach wird der erhaltene Rückstand aus 500 ml Acetonitril unter Verwendung von Aktivkohle umkristallisiert. Es werden 32.2 g der Titelverbindung als farbloser Feststoff mit einem Schmelzpunkt von 187.4 °C erhalten (MS: 365 (MH⁺), TLC: Rf = 0.29 (Cyclohexan/Methyl-tert-butyl-ether 1:4, Volumenanteile).

### Ia. 2-Ethyl-4-hydroxy-3-methyl-benzoesäure-N'-[(S)-2-(3,5-difluorphenyl)-2-hydroxy-acetyl]-hydrazid

a) Chromatographische Racemat-Trennung (ohne Fig.):
   500 mg von Beispiel 1 (EMD 638683) werden zu je 4 Portionen in je 5 ml 2-Propanol und 5 ml n-Heptan in der Wärme gelöst. Anschließend werden die Lösungen an 2 seriellen Chiralpak AD (5x20cm) Chromatographiesäulen (Fa. Daicel) mit dem Lösemittelgemisch n-Heptan/2-Propanol (4:1, Volumenanteile; 0,8 ml/min) chromatographiert (Retentionszeit: 6,11 min). Die vereinten Produktfraktionen werden im Vakuum zur Trockne eingeengt, wobei 245 mg der Titelverbindung analysen- und enantiomerenrein als farbloser Feststoff mit einem Schmelzpunkt von 202,3°C erhalten werden. MS: 365 (M+H⁺), 382 (M+NH₄⁺); DC: R_{f} = 0,29 (Cyclohexan/Methyl-tert.-butylether 1:4, Volumenanteile); [α]²⁰_{D} = +30,4° (c = 0,0201 g/2 ml Methanol).
b) aus A2:
   Die Titelverbindung wird in analoger Weise aus 4-Benzyloxy-2-ethyl-3-methylbenzoesäure-N'-[(S)-2-(3,5-difluorphenyl)-2-hydroxy-acetyl]-hydrazid (A2) wie für Verbindung 3 beschrieben hergestellt.

### Ib. 2-Ethyl-4-hydroxy-3-methyl-benzoesäure-N'-[(R)-2-(3,5-difluorphenyl)-2-hydroxy-acetyl]-hydrazid

a) Chromatographische Racemat-Trennung (ohne Fig.):
   500 mg von Beispiel 1 (EMD 638683) werden zu je 4 Portionen in je 5 ml 2-Propanol und 5 ml n-Heptan in der Wärme gelöst. Anschließend werden die Lösungen an 2 seriellen Chiralpak AD (5x20cm) Chromatographiesäulen (Fa. Daicel) mit dem Lösemittelgemisch n-Heptan/2-Propanol (4:1, Volumenanteile; 0,8 ml/min) chromatographiert (Retentionszeit: 9,36 min). Die vereinten Produktfraktionen werden im Vakuum zur Trockne eingeengt, wobei 250 mg der Titelverbindung als farbloser Feststoff mit einem Schmelzpunkt von 203,7°C erhalten werden. MS: 365 (M+H⁺); DC: Rf = 0,29 (Cyclohexan/Methyl-tert.-butylether 1:4, Volumenanteile); [α]²⁰_{D} = -29,7° (c = 0,0184g/2 ml Methanol), Enantiomerenüberschuss 98,2 %.
b) aus A3:
   45 mg 4-Benzyloxy-2-ethyl-3-methyl-benzoesäure-N'-[(R)-2-(3,5-difluorphenyl)-2-hydroxy-acetyl]-hydrazid (A3) werden in 100 ml Methanol gelöst. Anschließend wird die Reaktionslösung am H-Cube® (Fa. ThalesNano) an 10% Pd/C (55 x 4 mm Kartusche) hydriert (Fluss: 1 ml/min, Modus: *full H*₂, 30°C, Normaldruck). Anschließend wird die Reaktionslösung zur Trockne eingeengt und mittels präparativer HPLC (Chromolith® prep RP18e 100-25 mm, Fa. Merck; Lösemittelgradient: Wasser / 1-50 vol.% Acetonitril) aufgereinigt, wobei 10 mg der Titelverbindung nach Gefriertrocknung als amorphes, farbloses Lyophilisat erhalten werden; MS: 751,2 (2M+Na⁺); DC: Rf = 0,31 (Cyclohexan/Methyl-tert.-butylether 1:4, Volumenanteile), Enantiomerenüberschuss >50 %.

### Zwischenverbindungen

### A1. 2-Ethyl-4-methoxy-3-methyl-benzoesäure-N'-[2-(3,5-difluor-phenyl)-2-hydroxy-acetyl]-hydrazid

Es werden 106.0 g 3,5-difluor-phenyl-hydroxy-essigsäurehydrazid (B2) in 820 ml N,N-Dimethylformamid gelöst. Anschließend werden 104.9 g 2-Ethyl-4-methoxy-3-methyl-benzoesäure (B1), 153.9 g N-3-dimethylaminopropyl-N'-ethylcarbodiimidhydrochlorid (DAPECI) und 42.8 g Hydroxybenzotriazol (HOBt) hinzugegeben. Die Reaktionsmischung wird während der Zugabe der vorstehend aufgezählten Verbindungen in einem Eisbad gekühlt. Danach wird die Lösung bei Raumtemperatur während 24 Stunden gerührt. Zur Aufarbeitung wird die Reaktionsmischung mit 3,5 Liter Wasser verdünnt. Die erhaltene Suspension wird während einer Stunde gerührt, anschließend abfiltriert und mit zusätzlichem Wasser gewaschen. Der Filterkuchen wird aus 2,5 Liter Methanol umkristallisiert, wobei 129.0 g der Titelverbindung als farbloser Feststoff mit einem Schmelzpunkt von 197.1 °C erhalten werden (MS: 401 (MNa⁺)).

### A2. 4-Benzyloxy-2-ethyl-3-methyl-benzoesäure-N'-[(S)-2-(3,5-difluorphenyl)-2-hydroxy-acetyl]-hydrazid

Die Titelverbindung wird in analoger Weise unter Verwendung von (S)-Methyloxazaborolidin [(S)-Me-CBS] wie für das Zwischenprodukt A3 beschrieben hergestellt.

### A3. 4-Benzyloxy-2-ethyl-3-methyl-benzoesäure-N'-[(R)-2-(3,5-difluorphenyl)-2-hydroxy-acetyl]-hydrazid

302 mg 4-Benzyloxy-2-ethyl-3-methyl-benzoesäure-N'-[2-(3,5-difluorphenyl)-2-oxo-acetyl]-hydrazid (B3) werden in 25 ml Tetrahydrofuran gelöst. Diese Lösung wird binnen 5 min zu einer Lösung aus 49 µl Borandimethylsulfid-Komplex und 55 µl (R)-Methyloxazaborolidin [(R)-Me-CBS, 1-1,5 M in Toluol) in 17 ml Tetrahydrofuran bei einer Temperatur von (-)78°C zugetropft. Anschließend wird das Kältebad entfernt und die Lösung für 6 h gerührt, wobei die Reaktionslösung auf Raumtemperatur erwärmt. Danach werden 100 ml Wasser zugegeben und dreimal mit je 150 ml Essigsäureethylester extrahiert. Die vereinigten organischen Phasen werden mit je 40 ml Wasser, 0,1 M HCl und ges. NaCl-Lösung gewaschen, über NaSO4 getrocknet, abgesaugt und zur Trockne eingeengt. Der Rückstand wird mittels präparativer HPLC (Lösemittelgradient: Wasser / 1-50 vol.% Acetonitril) aufgereinigt, wobei 63 mg der Titelverbindung als farbloser, amorpher Feststoff erhalten werden. MS: 932 (2M+Na⁺); DC: Rf = 0,56 (Cyclohexan/Methyl-tert.-butylether 1:4, Volumenanteile).

### B1. 2-Ethyl-4-methoxy-3-methyl-benzoesäure

Es werden 20.0 g von 4-Methoxy-2,3-dimethyl-benzoesäure (C1) in einem Liter Tetrahydrofuran in einer trockenen Stickstoffatmosphäre gelöst und anschließend auf -78°C gekühlt. Danach werden 300 ml sec.-Butyllithium (1.4 M in Cyclohexan) tropfenweise unter Erhalt einer roten Lösung hinzugegeben. Die rote Lösung wird für eine weitere Stunde bei -78°C gerührt. Danach werden 34.5 ml Methyliodid hinzugegeben und die erhaltene Suspension wird ohne Kühlung während einer Stunde gerührt. Anschließend werden 300 ml Wasser hinzugefügt und die Lösung wird unter Verwendung von Ethylacetat zweimal mit je 300 ml Ethylacetat extrahiert. Anschließend wird die wässrige Phase filtriert, das Filtrat in einem Eisbad gekühlt, durch Zugabe von Salzsäure (3.0 M) auf pH 1 eingestellt und für weitere 30 Minuten gerührt. Danach wird die Suspension filtriert und mit Wasser gewaschen. Der Filterkuchen wird im Vakuum bei 110°C getrocknet, wobei 20.9 g der Titelverbindung als farbloser Feststoff mit einem Schmelzpunkt von 180 °C erhalten werden (MS: 195.2 (MH⁺)).

### B2. 3,5-Difluor-phenyl-hydroxy-essigsäurehydrazid

Es werden 5.0 g 3,5-difluor-phenyl-hydroxy-essigsäuremethylester (C2) in 45.0 ml 2-Propanol gelöst. Danach werden 1.32 ml Hydrazinhydrat tropfenweise hinzugegeben und die Reaktionsmischung während 18 Stunden unter Rückfluss gehalten. Anschließend wird die Lösung zur Trockene eingeengt und der erhaltene Rückstand durch Flash-Chromatographie auf Kieselgel gereinigt (Gradient zur Eluierung: Ethylacetat/ 0-20.0 Vol.% Methanol), wobei 3.70 g der Titelverbindung als farbloser Feststoff mit einem Schmelzpunkt von 119.3 °C erhalten werden (MS: 202.8 (MH⁺)).

### B3. 4-Benzyloxy-2-ethyl-3-methyl-benzoesäure-N'-[2-(3,5-difluorphenyl)-2-oxo-acetyl-hydrazid

172 mg 4-Benzyloxy-2-ethyl-3-methyl-benzoesäure-N'-[2-(3,5-difluorphenyl)-2-hydroxy-acetyl]-hydrazid (C3) werden in 0,9 ml Dimethylsulfoxid und 28 ml Dichlormethan unter einer trockenen Stickstoff-Atmosphäre gelöst. Anschließend werden 185 mg Triacetoxy-Perjodinan (Dess-Martin Reagenz) zugegeben. Die Suspension wird 1 h bei Raumtemperatur gerührt. Zur Aufarbeitung werden 100 ml Dichlormethan und 30 ml 0,5M Na₂S₂O₃-Lösung zugegeben. Die organische Phase wird anschließend mit 50 ml ges. NaHCO₃-Lösung extrahiert, über Na₂SO₄ getrocknet, abgesaugt und zur Trockne eingeengt. Der Rückstand wird mittels Flashsäulenchromatographie an Kieselgel (Lösemittelgradient: Cyclohexan / 0-30 vol.% Methyl-tert.-butylether) aufgereinigt, wobei 163 mg der Titelverbindung als amorpher Feststoff erhalten werden; MS: 452,7 (2M+H⁺); DC: Rf = 0,37 (Cyclohexan/Essigsäureethylester 2:1, Volumenanteile).

### C1. 4-Methoxy-2,3-dimethylbenzoesäure

Es werden 60.0 g 2,3-Dimethyl-4-methoxybenzaldehyd (D1) in 1.20 I Dimethylsulfoxid gelöst. Anschließend wird eine Lösung von 131.5 g Natriumdihydrogenphoshat und 132.2 g Natriumchlorit in 480 ml Wasser tropfenweise hinzugegeben, wobei die Temperatur des Reaktionsansatzes unterhalb von 40 °C gehalten wird. Danach wird die Reaktionsmischung für weitere 2 Stunden bei Raumtemperatur gerührt. Zur Aufarbeitung wird die Suspension mit 2.0 I Wasser verdünnt und für eine weitere Stunde gerührt. Der erhaltene Niederschlag wird filtriert, mit Wasser gewaschen und im Vakuum bei 100 °C getrocknet. Es werden 65.0 g der Titelverbindung als farbloser Feststoff mit einem Schmelzpunkt von 214 °C erhalten (MS: 181.2 (MH⁺)).

### C2. 3,5-Difluor-phenyl-hydroxy-essigsäuremethylester

Es werden 25.0 g 3,5-Difluor-phenyl-hydroxy-essigsäure (D2) in 325 ml Methanol gelöst. Anschließend werden 3,9 g Borsäure hinzugegeben und die Reaktionsmischung während 18 Stunden bei Raumtemperatur gerührt. Danach wird die erhaltene Lösung zur Trockene eingeengt. Der Rückstand wird unter Verwendung von 100 ml Ethylacetat und 100 ml Wasser extrahiert. Die wässrige Phase wird anschließend zweimal mit je 50 ml Ethylacetat extrahiert.

Die vereinigten organischen Phasen werden über Natriumsulfat getrocknet, und filtriert. Das Filtrat wird im Vakuum zur Trockne eingeengt, wobei 25.5 g der Titelverbindung als Rohprodukt erhalten. Umkristallisation aus 17 ml Ethylacetat und 170 ml Cyclohexan ergeben 23.5 g der reinen Verbindung C2 mit einem Schmelzpunkt von 61.8 °C (MS 201.9 (M⁺)).

### C3. 4-Benzyloxy-2-ethyl-3-methyl-benzoesäure-N'-[2-(3,5-difluorphenyl)-2-hydroxy-acetyl]-hydrazid

Verbindung C3 wird in analoger Weise der zu A1 beschriebenen Herstellung ausgehend von 4-Benzyloxy-2-ethyl-3-methyl-benzoesäure und Zwischenprodukt B2 synthetisiert.

### D1. 2,3-Dimethyl-4-methoxybenzaldehyd

Die Verbindung D1 wurde von ABCR GmbH & Co. KG bezogen.

### D2. 3,5-Difluor-Phenyl-hydroxy-essigsäure

Die Verbindung D2 wurde von Sigma-Aldrich Co. bezogen.

### Biochemischer Assay

Die in den Beispielen beschriebenen erfindungsgemäßen Verbindungen wurden in dem unten beschriebenen Assay geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).
Die Hemmung der SGK1 Proteinkinase kann im Filterbindungsverfahren bestimmt werden.

### Biochemische Daten

| Verbindung Nr. | SGK1 Enzyminhibierung |
|---|---|
| | IC₅₀ |
| I | > 300 nM |
| Ia | > 300 nM |
| Ib | < 300nM |

### Figurenbeschreibung

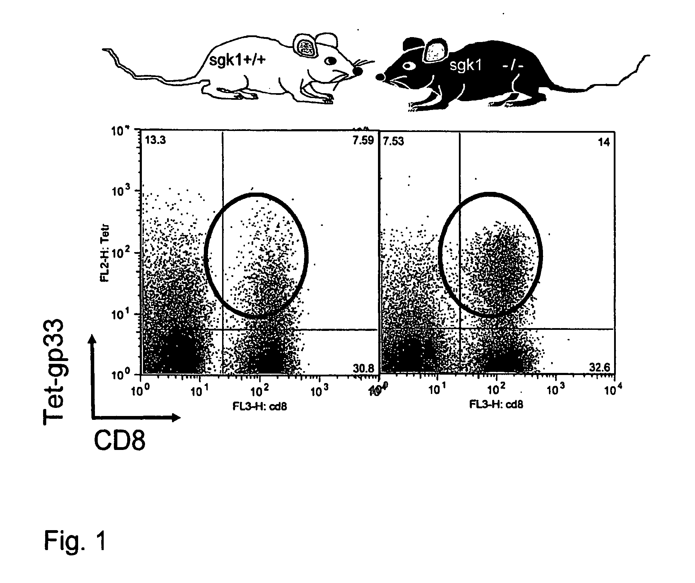

In Figur 1 ist ein Dot-Plot von virusspezifischen (Tet-9P 33⁺) CD8⁺T-Lymphozyten im Blut von SGK1-Knockout-Mäusen (SGK1^{-/-}, rechtes Dot-Plot) und ihren Wildtyp-Geschwistermäusen (SGK1^{+/+}, linkes Dot-Plot) dargestellt. Hierzu wurden SGK1-Knockout-Mäuse und Wildtyp-Geschwister Mäuse jeweils mit 2x 10⁶ pfu (plaque forming units) LCMV (Choriomeningitis-Virus) infiziert. Am 8. Tag nach der Infektion wurde das Blut mit Hilfe der sogenannten Tetramer-Technik für virusspezifische CD8⁺ T-Zellen gefärbt und anschließend die virusspezifischen CD8⁺ T-Zellen mit Hilfe der FACS-Analyse (fluorescence activated cell sorting, Durchflusszytometrie) identifiziert und quantifiziert. Die in Figur 1 dargestellten Dot-Plots dokumentieren eine gesteigerte Immunabwehr gegenüber LCMV bei den SGK1-Knockout-Mäusen.

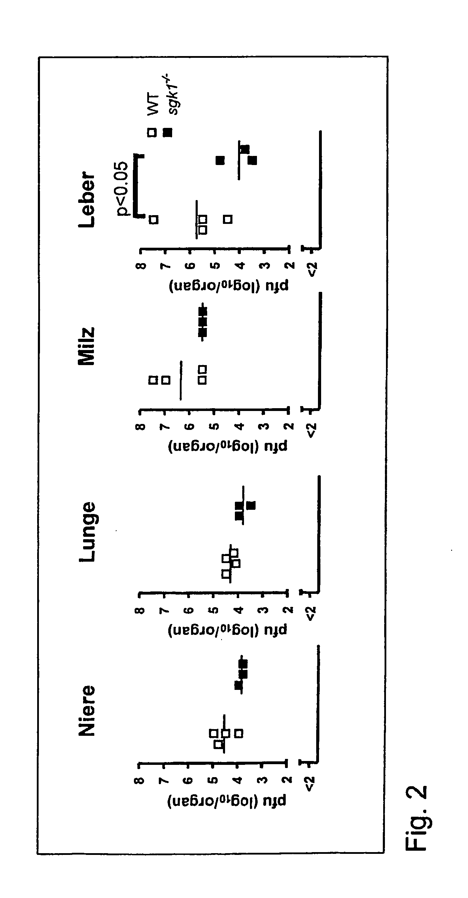

Figur 2 gibt graphisch die Anzahl von Plaque bildenden Einheiten von LCMV infizierten SGK1-Knockout-Mäusen (SGK1^{-/-}, geschlossene Quadrate) und ihren Wildtyp-Geschwistermäusen (VVT, offene Quadrate) in verschiedenen Geweben wieder. Hierzu wurden die SGK1-Knockout-Mäuse sowie die Wildtyp-Geschwistermäuse mit jeweils 2x 10⁶ pfu (plaque forming units) LCMV infiziert. Am 8. Tag nach der Infektion wurden die Virustiter mit Hilfe des sogenannten plug assays in den verschiedenen Geweben (Nieren-, Lungen-, Milz- und Lebergewebe) analysiert. Auf der Ordinate der in Figur 2 dargestellten Graphiken ist jeweils der dekadische Logarithmus der Anzahl der Plaque bildenden Einheiten (pfu, plaque forming units) aufgetragen. Die in Figur 2 wiedergegebenen Graphiken zeigen, dass bei den SGK1-Knockout-Mäusen die Anzahl der Plaque bildenden Einheiten von LCMV in den verschiedenen Gewebetypen geringer ausfällt als bei den Wildtyp-Geschwistermäusen.

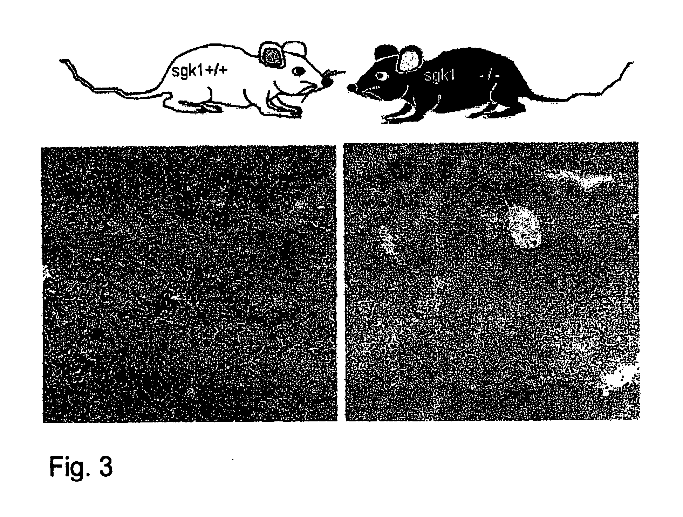

Figur 3 zeigt das Ergebnis einer immunohistochemischen Untersuchung auf LCMV-Antigen bei SGK1-Knockout-Mäusen (SGK1^{-/-}, rechts) und Wildtyp-Geschwistermäusen (links). Hierzu wurden SGK1-Knockout-Mäuse und Wildtyp-Geschwistermäuse jeweils mit 2x 10⁶ pfu LCMV infiziert. Anschließend wurde am 8. Tag nach der Infektion Lebergewebe von SGK1-Knockout-Mäusen und von Wildtyp-Geschwistermäusen immunohistochemisch auf LCMV-Antigen untersucht.

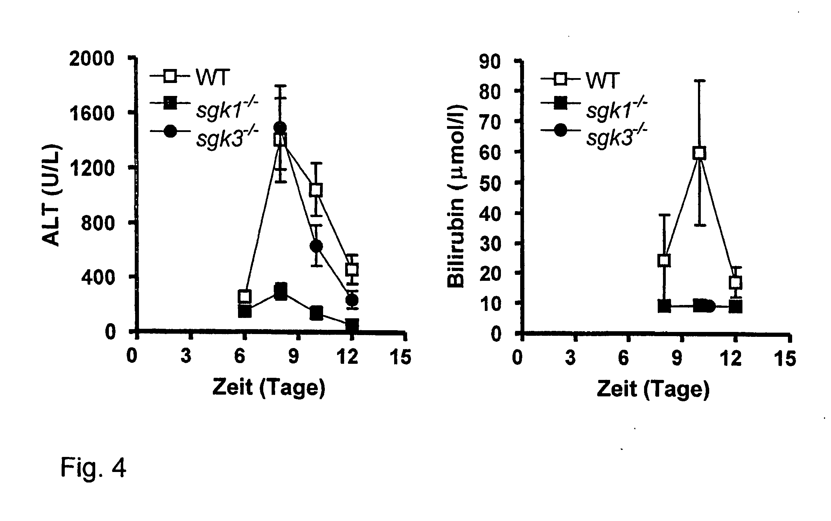

Figur 4 zeigt den Einfluss von SGK1 auf zwei typische Hepatitis-Marker, nämlich das Leberenzym Alanin-Aminotransferase (linke Graphik) und Bilirubin (rechte Graphik). Beide Marker kommen bei einer Hepatitis vermehrt im Lebergewebe vor.

Auf der Ordinate der linken Graphik von Figur 4 ist die Konzentration von Alanin-Aminotransferase (ALT), ausgedrückt in Einheiten pro Liter Gewebeflüssigkeit (U/L), wiedergegeben. Auf der Abszisse ist die Zeit in Tagen wiedergegeben. Auf der Ordinate der rechten Graphik von Figur 4 ist die Bilirubinkonzentration, ausgedrückt in µmol pro Liter Gewebeflüssigkeit, aufgetragen. Auf der Abszisse ist ebenfalls die Zeit, ausgedrückt in Tagen, wiedergegeben.

Die in Figur 4 dargestellten Graphiken zeigen, dass die Konzentrationen von Alanin-Aminotransferase und Bilirubin bei einer LCMV induzierten Hepatitis in SGK1-Knockout-Mäusen (SGK1^{-/-}, geschlossene Quadrate) im Verhältnis zu den entsprechenden Werten in Wildtyp-Geschwistermäusen (WT, offene Quadrate) deutlich erniedrigt sind. Diese Daten belegen, dass sich ein "Ausschalten" oder eine Hemmung bzw. Inhibierung von SGK1 unter therapeutischen Gesichtspunkten günstig auf den Verlauf der viralen Hepatitis auswirkt. Die in Figur 4 dargestellten Graphiken zeigen darüber hinaus auch, dass SGK3 keine den Verlauf einer viralen Hepatitis begünstigende Wirkung entfaltet (vgl. hierzu die in den Graphiken der Figur 4 abgebildeten geschlossenen Kreise, welche die entsprechenden Werte von SGK3-Knockout-Mäusen (SGK3^{-/-}) widerspiegeln). Insgesamt wurden auch in diesem Fall die SGK1-Knockout-Mäuse und die Wildtyp-Geschwistermäuse (sowie die SGK3-Knockout Mäuse) jeweils mit 2x 10⁶ pfu LCMV infiziert.

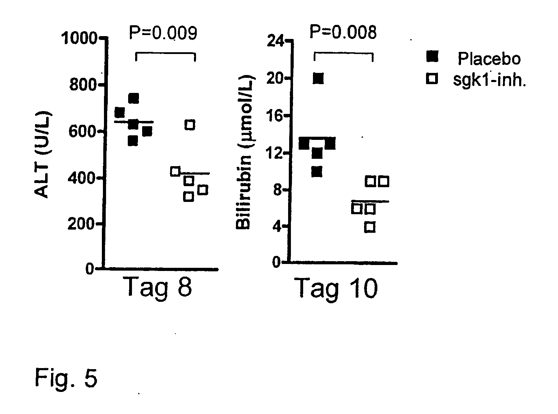

Die Figur 5 zeigt graphisch den Einfluss des SGK1-Inhibitors 2-Ethyl-4-hydroxy-3-methyl-benzoesäure-N'-[2-(3,5-difluor-phenyl)-2-hydroxy-acetyl]-hydrazid auf die Konzentration von Alanin-Aminotransferase sowie von Bilirubin. SGK1-Wildtyp-Mäuse vom Typ 10 C57BL/6 wurden mit Placebo-Futter gefüttert. Nach einer Woche wurde eine Gruppe der Versuchstiere mit dem SGK1-Inhibitor behandelt. Der SGK1-Inhibitor war dem Futter in einem Mischungsverhältnis von 4.46 mg/g Futter beigemischt worden. Dies entspricht einer ungefähren Dosis von 600 mg/kg Körpergewicht. Der anderen Gruppe wurde weiterhin lediglich das Placebo-Futter verabreicht. Nach einer weiteren Woche wurden die SGK1-Knockout-Mäuse jeweils mit 2x 10⁶ pfu LCMV infiziert, um auf diese Weise eine virale Hepatitis zu induzieren. Die Werte für die Alanin-Aminotransferase wurden 8 Tage nach der Infektion und die entsprechenden Werte für Bilirubin 10 Tage nach der Infektion gemessen. Auf der Ordinate der linken Graphik von Figur 5 ist die Konzentration von Alanin-Aminotransferase wiedergegeben (U/I, Enzymeinheiten pro Liter Gewebeflüssigkeit). Auf der Abszisse ist der Zeitraum von 8 Tagen nach der LCMV-Infektion dargestellt. Auf der Ordinate der rechten Graphik von Figur 5 ist die Bilirubinkonzentration (µmol/L) abgebildet. Die Abszisse zeigt den Zeitraum von 10 Tagen nach der LCMV-Infektion an.

Die in den Graphiken von Figur 5 wiedergegebenen Ergebnisse demonstrieren, dass die Konzentrationen der Hepatitis-Marker Alanin-Aminotransferase und Bilirubin bei den Versuchstieren, welche vor der Infektion mit dem SGK1-Inhibitor behandelt wurden (offene Quadrate), im Gegensatz zu den nicht mit dem SGK1-Inhibitor behandelten Versuchstieren (geschlossene Quadrate) deutlich erniedrigt sind. Diese Daten belegen, dass der SGK1-Inhibitor den Verlauf von viraler Hepatitis begünstigende Eigenschaften aufweist und damit zur Prophylaxe bzw. Behandlung von viraler Hepatitis eingesetzt werden kann.

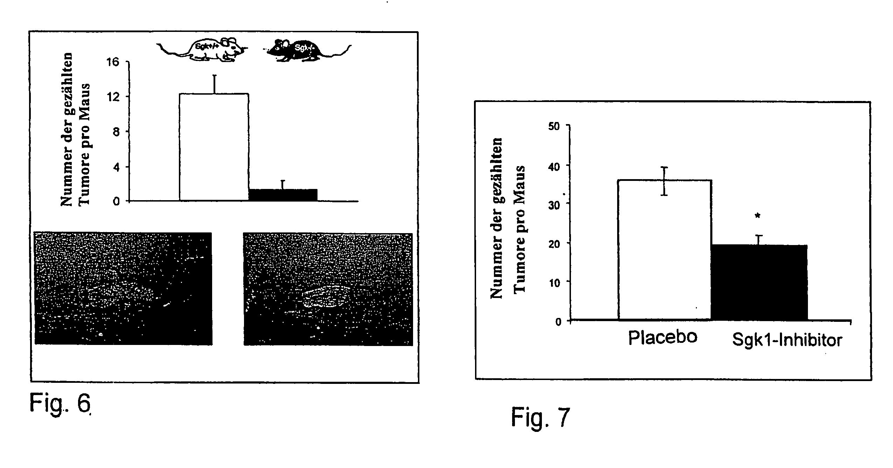

Figur 6 zeigt die Entwicklung von Colonkarzinomen bei SGK1-Knock-out Mäusen (SGK1^{-/-}-Mäusen) im Vergleich zu SGK1-Wildtyp-Mäusen (SGK^{+/+}-Mäusen). Hierzu wurden in den Versuchstieren Colontumore chemisch induziert (Wang JG, Wang DF, Lv BJ, Si JM: A novel mouse model for colitis-associated colon carcinogenesis induced by 1,2-dimethylhydrazine and dextran sulfate sodium. World J Gastroenterol 2004; 10:2958-2962). Ein Teil der Versuchstiere wurde in einem Alter von 8 Wochen drei Zyklen einer chemischen Tumorgenese ausgesetzt. Dazu wurden die Tiere intraperitoneal mit 20 mg/kg 1,2-Dimethylhydrazin (DMH; Sigma-Aldrich Corporation. St. Louis. MO. USA) behandelt. Beginnend eine Woche später wurde den Tieren drei Mal für 7 Tage destilliertes Wasser mit 30 g/l synthetischem Dextransulfatnatrium (DSS; Molmasse 5000; Wako Pure Chemical Industries Ltd. Japan) und für weitere 14 Tage destilliertes Wasser verabreicht (Dauer insgesamt 3 x (1 + 2 Wochen) = 9 Wochen insgesamt). Der andere Teil der Versuchstiere (Kontrolltiere) wurde stattdessen intraperitoneal per Injektion mit 20 mg/kg einer 0,9 %igen Natriumchlorid-Lösung versorgt. Alle Mäuse wurden im Alter von 22 Wochen mit Äther anästhesiert und anschließend getötet.

Das in Figur 6 graphisch dargestellte Ergebnis der chemisch induzierten Tumorgenese zeigt deutlich, dass die Empfindlichkeit von SGK1-Knockout-Mäusen (wiedergegeben durch den rechten geschlossenen Balken) im Gegensatz zu SGK1-Wildtyp-Mäusen (wiedergegeben durch den linken offenen Balken) gegen chemisch induzierte Colontumore signifikant herabgesetzt ist. Dieses Ergebnis ist um so überraschender, als die Fachliteratur bezüglich verschiedener Karzinomtypen von einer Herabregulierung von SGK1 in karzinomatösem Gewebe berichtet (Rauhala HE, Porkka KP, Tolonen TT, Martikainen PM, Tammela TL, Visakorpi T: Dualspecificity phosphatase 1 and serum/glucocorticoid-regulated kinase are downregulated in prostate cancer. Int J Cancer 2005; Chu S, Rushdi S, Zumpe ET, Mamers P, Healy DL, Jobling T, Burger HG, Fuller PJ: FSH-regulated gene expression profiles in ovarian tumours and normal ovaries. Mol Hum Reprod 2002; 8:426-433; Chung EJ, Sung YK, Farooq M, Kim Y, Im S, Tak WY, Hwang YJ, Kim YI, Han HS, Kim JC, Kim MK: Gene expression profile analysis in human hepatocellular carcinoma by cDNA microarray. Mol Cells 2002; 14:382-387), weswegen eigentlich zu erwarten gewesen wäre, dass eine Hemmung von SGK1 das Tumorwachstum fördert.

Figur 7 zeigt graphisch die Wirkung des SGK1-Inhibitors 2-Ethyl-4-hydroxy-3-methyl-benzoesäure-N'-[2-(3,5-difluor-phenyl)-2-hydroxy-acetyl]-hydrazid auf die Entstehung von Colonkarzinomen. Zunächst wurde eine wie in der Figurenbeschreibung zu Figur 6 beschriebene chemisch induzierte Tumorgenese durchgeführt. Eine Woche nach der Behandlung wurde für 20 Tage entweder ein Placebo oder der SGK1-Inhibitor verabreicht. Der SGK1-Inhibitor war dem Futter in einem Mischungsverhältnis von 4.46 mg/g Futter beigemischt worden. Dies entspricht einer ungefähren Dosis von 600 mg/kg Körpergewicht. Die in Figur 7 dargestellte Graphik verdeutlicht, dass im Falle der mit dem SGK1-Inhibitor behandelten Versuchstiere das Wachstum von Colontumoren (rechter geschlossener Balken) im Vergleich zu den unbehandelten Versuchstieren (linker offener Balken) signifikant gehemmt wird.

## Patentansprüche

1. Verbindung der Formel Ia und Ib, insbesondere zur Verwendung als Arzneimittel: 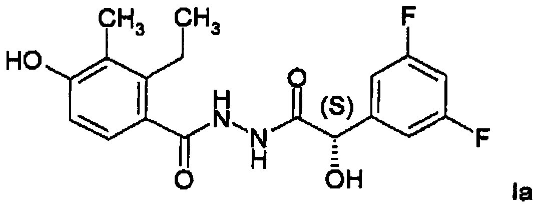 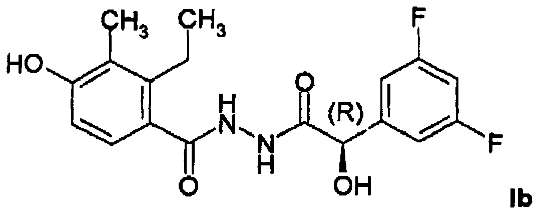 sowie ihre pharmazeutisch verwendbaren Tautomere, Salze, und die Enantiomeren, einschließlich deren Mischungen in allen Verhältnissen.

2. Verbindungen nach Anspruch 1 zur Behandlung von viralen Erkrankungen, **dadurch gekennzeichnet, dass** die viralen Erkrankungen aus der Gruppe lymphozytäre Choriomeningitis, virale Hepatitis, virale Myocarditis, Aids, Herpes, Papilloma und virale Lungenentzündung ausgewählt sind.

3. Verbindungen nach Anspruch 1, zur Behandlung von Karzinomen, **dadurch gekennzeichnet, dass** die Karzinome aus der Gruppe Colonkarzinom, Mammakarzinom, Magenkarzinom und Lungenkarzinom ausgewählt sind.

4. Verwendung von Verbindungen nach einem der vorhergehenden Ansprüche zur Herstellung eines Arzneimittels zur Prophylaxe und/oder Behandlung von viralen Erkrankungen und/oder Karzinomen.

## Claims

1. Compounds of the formulae Ia and Ib, in particular for use as medicaments: 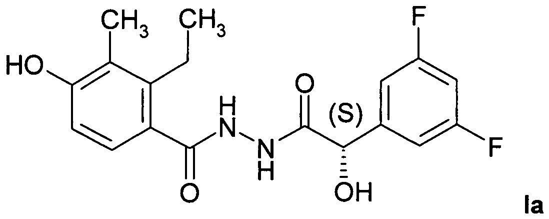 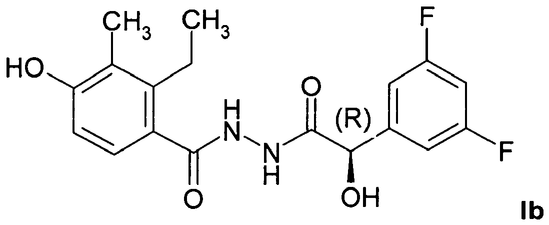 and their pharmaceutically usable tautomers, salts, and the enantiomers, including mixtures thereof in all ratios.

2. Compounds according to Claim 1 for the treatment of viral diseases, **characterised in that** the viral diseases are selected from the group lymphocytic choriomeningitis, viral hepatitis, viral myocarditis, AIDS, herpes, papilloma and viral lung inflammation.

3. Compounds according to Claim 1 for the treatment of carcinomas, **characterised in that** the carcinomas are selected from the group colon carcinoma, mammacarcinoma, stomach carcinoma and lung carcinoma.

4. Use of compounds according to one of the preceding claims for the preparation of a medicament for the prophylaxis and/or treatment of viral diseases and/or carcinomas.

## Revendications

1. Composés de formules la et Ib, en particulier pour une utilisation comme médicaments : 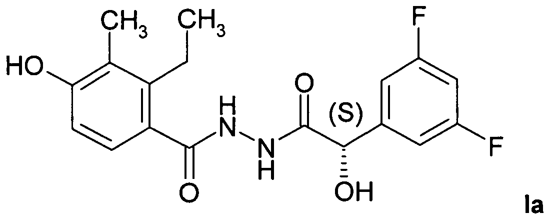 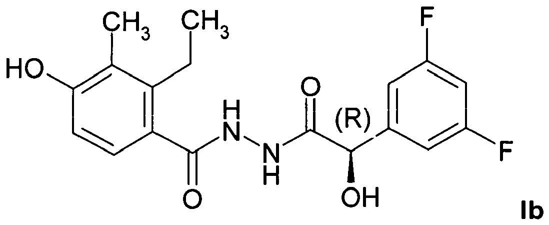 et leurs tautomères et sels pharmaceutiquement utilisables, et leurs énantiomères, y compris des mélanges de ceux-ci en toutes proportions.

2. Composés selon la revendication 1, pour le traitement de maladies virales, **caractérisés en ce que** les maladies virales sont choisies dans le groupe constitué par la chorioméningite lymphocytaire, l'hépatite virale, la myocardite virale, le SIDA, l'herpès, le papillome et les inflammations pulmonaires virales.

3. Composés selon la revendication 1, pour le traitement de carcinomes, **caractérisés en ce que** les carcinomes sont choisis dans le groupe constitué par le carcinome du côlon, le carcinome mammaire, le carcinome de l'estomac et le carcinome des poumons.

4. Utilisation de composés selon l'une des revendications précédentes, pour la préparation d'un médicament destiné à la prophylaxie et/ou au traitement de maladies virales et/ou de carcinomes.
